# EUROPÄISCHE PATENTSCHRIFT

(11) **EP 2 408 571 B1**
(45) Veröffentlichungstag und Bekanntmachung des Hinweises auf die Patenterteilung: **16.09.2015**
(21) Anmeldenummer: 10710790.6
(22) Anmeldetag: 12.03.2010
(51) Int. Cl.: B05B 11/00, B65D 83/14, A61J 1/10

(54) **RESERVOIR UND ZERSTÄUBER**
RESERVOIR AND ATOMIZER
RESERVOIR ET PULVERISATEUR

(30) Priorität: 17.03.2009 EP 09003818
(43) Veröffentlichungstag der Anmeldung: 25.01.2012
(73) Patentinhaber: Boehringer Ingelheim International GmbH, 55216 Ingelheim am Rhein (DE)
(72) Erfinder: HAUSMANN, Matthias, 55216 Ingelheim am Rhein (DE); HENNING, Carsten, 55216 Ingelheim am Rhein (DE); KLADDERS, Heinrich, 55216 Ingelheim am Rhein (DE)
(74) Vertreter: Simon, Elke Anna Maria
(86) Internationale Anmeldenummer: PCT/EP2010/001573
(87) Internationale Veröffentlichungsnummer: WO 2010/105775

(56) Entgegenhaltungen:
- EP-A1- 1 223 115
- WO-A1-99/43571
- WO-A2-00/49988

## Beschreibung

Die vorliegende Erfindung betrifft ein Reservoir gemäß dem Oberbegriff des Anspruchs 1.

Aus der WO 99/43571 A1 ist ein Behälter für einen Zerstäuber bzw. Inhalator bekannt. Der Behälter weist eine starre metallische Außenhülle und einen darin aufgenommenen Beutel auf. Der Beutel bildet einen Fluidraum für eine Arzneimittelzubereitung und kollabiert, wenn die Arzneimittelzubereitung entnommen wird. Der Beutel ist durch einen vorzugsweise zweiteiligen, insbesondere flanschartigen Verschluß verschlossen. Ein erstes Verschlußteil wird fest mit dem Beutel verbunden. Nach dem Einfüllen der Arzneimittelzubereitung durch eine Öffnung des ersten Verschlußteils hindurch wird ein zweites Verschlußteil mit dem ersten Verschlußteil vorzugsweise durch Ultraschallschweißen verbunden, um die Arzneimittelzubereitung bzw. den Fluidraum hermetisch zu verschließen. Zur Entnahme der Arzneimittelzubereitung kann der Verschluß bzw. das zweite Verschlußteil von einem Förderelement durchstochen werden, das sich dann in den Fluidraum hinein erstreckt.

Bei dem bekannten Behälter besteht das potentielle Problem, daß es insbesondere beim Ultraschallverschweißen des zweiten Verschlußteils mit dem ersten Verschlußteil zur Entstehung von Schweißpartikeln (Austrieb) kommen kann. Diese Schweißpartikel können in das Innere des Behälters bzw. in den Fluidraum oder in die Arzneimittelzubereitung eindringen.

Der vorliegenden Erfindung liegt die Aufgabe zugrunde, ein Reservoir mit einem Fluidraum für ein Fluid, insbesondere für eine Arzneimittelzubereitung, sowie einen Zerstäuber oder Inhalator mit einem derartigen Reservoir anzugeben, wobei auf möglichst einfache und effiziente Weise verhindert werden kann, daß beim Verschließen des Fluidraums durch Verbinden von zwei Verschlußteilen, insbesondere durch Verschweißen der Verschlußteile, potentiell auftretende Partikel oder sonstige Verschmutzungen in das Fluid bzw. den Fluidraum eindringen.

Die obige Aufgabe wird durch ein Reservoir gemäß Anspruch 1 oder einen mit diesem Reservoir versehenen Zerstäuber gelöst. Vorteilhafte Weiterbil. dungen sind Gegenstand der Unteransprüche.

Ein Aspekt der vorliegenden Erfindung liegt darin, daß der Verschluß des Reservoirs bzw. Fluidraums eine fluidseitig bezüglich eines Verbindungsbereichs der beiden Verschlußteile angeordnete Sperre zum Verhindern des Eindringens von bei Verbinden der Verschlußteile potentiell entstehenden Partikeln oder sonstigen Verschmutzungen in das Fluid und/oder in den Fluidraum aufweisen. Insbesondere ist die Sperre durch einen Preßsitz und/oder Auflagebereich der beiden Verschlußteile gebildet. So kann auf sehr einfache und effiziente Weise verhindert werden, daß beim Verbinden der beiden Verschlußteile potentiell entstehende Partikel oder sonstige Verschmutzungen in das Fluid und/oder in den Fluidraum eindringen.

Eine weitere Aufgabe der vorliegenden Erfindung liegt darin, ein Reservoir mit einem Fluidraum für ein Fluid, insbesondere für eine Arzneimittelzubereitung, sowie einen Zerstäuber oder Inhalator mit einem derartigen Reservoir anzugeben, wobei der Fluidraum bei einfacher Herstellbarkeit und/oder großem Aufnahmevolumen dicht ist und/oder sehr leicht kollabiert.

Ein auch unabhängig realisierbarer Aspekt der Erfindung liegt darin, daß der Fluidraum sich zu seinem freien Ende hin insbesondere biberschwanzartig verbreitert. Dies ist einem leichtem Kollabieren bei der Entnahme von Fluid, einen großen Aufnahmevolumen und/oder einer einfachen Herstellung zuträglich.

Ein anderer, auch unabhängiger realisierbarer Aspekt der vorliegenden Erfindung liegt darin, daß der Fluidraum durch eine Wandung mit einer Längsnaht gebildet ist, bei der Längskanten eines die Wandung bildenden Materialstücks im wesentlichen auf Stoß durch einen Materialstreifen - besonders bevorzugt aus einem anderen und/oder zusätzlichem Material - miteinander verbunden sind. Dies ist einem leichten Kollabieren bei der Entnahme von Fluid und/oder einer einfachen Herstellung zuträglich.

Die vorgenannten Aspekte und Merkmale der vorliegenden Erfindung sowie die nachfolgend erläuterten Aspekte und Merkmale der vorliegenden Erfindung können unabhängig voneinander, aber auch in einer beliebigen Kombination realisiert werden.

Weitere Vorteile, Merkmale, Eigenschaften und Aspekte der vorliegenden Erfindung ergeben sich aus den den Schutzbereich festlegenden Ansprüchen und der folgenden Beschreibung einer bevorzugten Ausführungsform anhand der Zeichnung. Es zeigt:
- Fig. 1: einen schematischen Schnitt eines vorschlagsgemäßen Zerstäubers im ungespannten Zustand, der ein vorschlagsgemäßes Reservoir enthält;
- Fig. 2: einen schematischen, um 90° gegenüber Fig. 1 gedrehten Schnitt des Zerstäubers im gespannten Zustand;
- Fig. 3: einen schematischen Schnitt des Reservoirs;
- Fig. 4: einen anderen schematischen Schnitt des Reservoirs;
- Fig. 5: eine ausschnittsweise Vergrößerung von Fig. 4;
- Fig. 6: einen Schnitt eines zweiten Verschlußteils eines Verschlusses des Reservoirs;
- Fig. 7a-k: schematische Darstellungen von verschiedenen Siegelgeometrien eines Fluidraums des Reservoirs; und
- Fig. 8a-c: schematische Querschnitte unterschiedlich hergestellter Fluidräume.

In den Figuren werden für gleiche oder ähnliche Teile dieselben Bezugszeichen verwendet, wobei insbesondere entsprechende oder vergleichbare Eigenschaften und Vorteile erreicht werden, auch wenn eine wiederholte Beschreibung weggelassen ist.

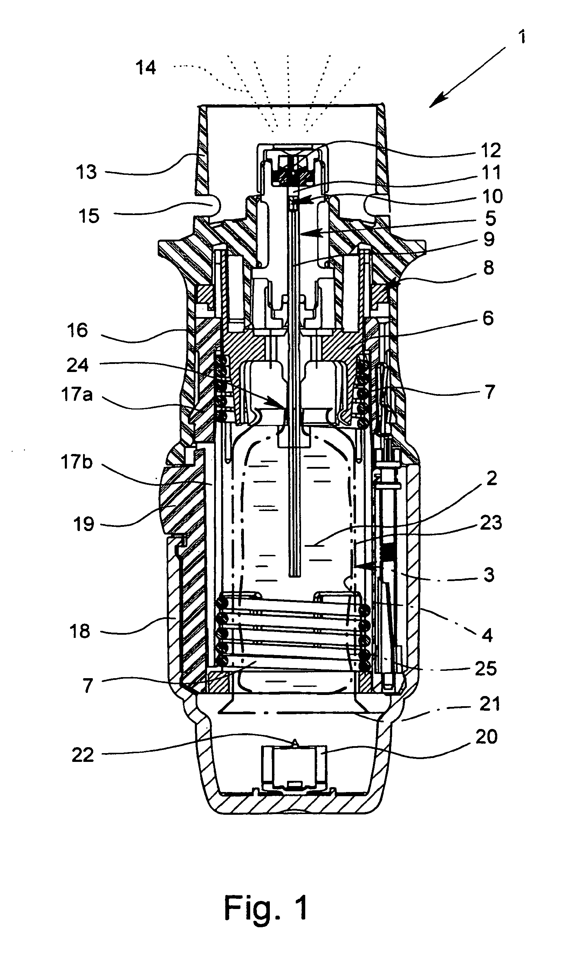

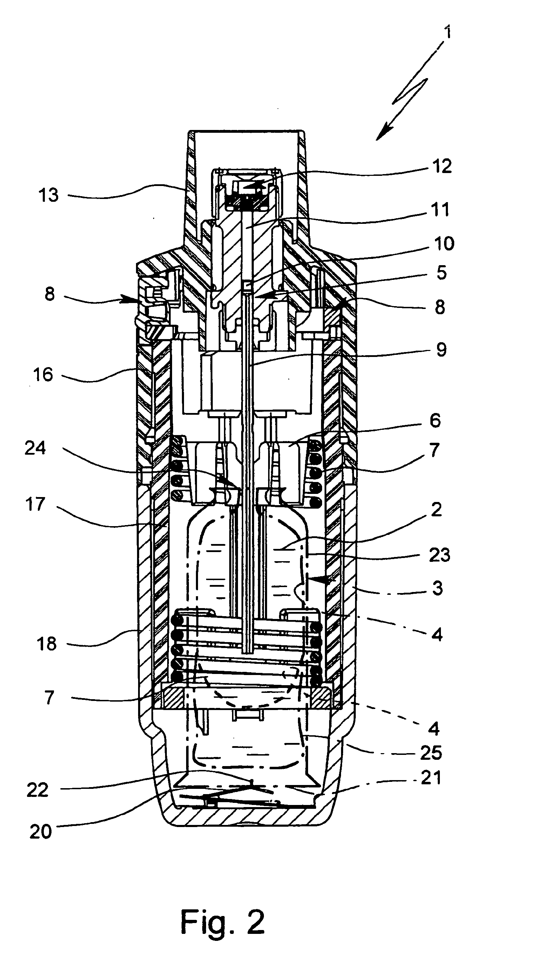

Fig. i und 2 zeigen einen vorschlagsgemäßen Zerstäuber 1 zur Zerstäubung eines Fluids 2, insbesondere einer Flüssigkeit bzw. Arzneimittelzubereitung, in einer schematischen Darstellung im ungespannten Zustand (Fig. 1) und gespannten Zustand (Fig. 2). Der Zerstäuber 1 ist insbesondere als tragbarer Inhalator ausgebildet und/oder arbeitet vorzugsweise ohne Treibgas.

Bei Zerstäubung des Fluids 2 bzw. der Arzneimittelzubereitung wird vorzugsweise ein lungengängiges Aerosol 14 (Fig. 1) gebildet, das von einem nicht dargestellten Benutzer bzw. Patienten aufgenommen, insbesondere eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der Zerstäuber 1 weist ein vorzugsweise einsetzbares und ggf. wechselbares Reservoir 3 mit dem Fluid 2 auf, wie in Fig. 1 und 2 gezeigt. Vorzugsweise enthält das Reservoir 3 eine ausreichende Menge (typischerweise 2 bis 10 oder 2 bis 15 ml) an Fluid 2 bzw. Wirkstoff für mehrere Dosen, um also mehrere Zerstäubungen oder Anwendungen zu ermöglichen.

Das Reservoir 3 ist vorzugsweise im wesentlichen zylindrisch bzw. kartuschenartig, länglich und/oder als insbesondere starrer Behälter ausgebildet und/oder beispielsweise von unten, nach Öffnen des Zerstäubers 1, in diesen einsetzbar und ggf. wechselbar.

Das Reservoir 3 weist einen vorzugsweise beutelartig oder schlauchartig ausgebildeten Fluidraum 4 mit dem Fluid 2 auf. Der Fluidraum 4 bzw. eine den Fluidraum 4 begrenzende Wandung 25 ist vorzugsweise - zumindest bereichsweise - flexibel, verformbar und/oder kollabierbar ausgebildet.

Der Zerstäuber 1 weist vorzugsweise eine Fördereinrichtung, insbesondere einen Druckerzeuger 5, zur Förderung und/oder Zerstäubung des Fluids 2, insbesondere jeweils in einer vorbestimmten, ggf. einstellbaren Dosiermenge auf.

Der Zerstäuber 1 bzw. Druckerzeuger 5 weist insbesondere eine Halterung 6 für das Reservoir 3, eine zugeordnete, nur teilweise dargestellte Antriebsfeder 7 vorzugsweise mit einem zugeordneten, zur Entsperrung manuell betätigbaren Sperrelement 8, ein vorzugsweise als Kapillare ausgebildetes Förderelement bzw. Förderrohr 9, ein optionales Ventil, insbesondere Rückschlagventil 10, eine Druckkammer 11 und/oder eine Austragsdüse 12 insbesondere im Bereich eines Mundstücks 13 oder sonstigen Endstücks auf.

Das Reservoir 3 wird über die Halterung 6, insbesondere klemmend oder rastend, so in dem Zerstäuber 1 fixiert, daß das Förderelement in den Fluidraum 4 eintaucht und/oder damit fluidisch verbunden wird. Die Halterung 6 kann dabei derart ausgebildet sein, daß das Reservoir 3 ausgetauscht werden kann.

Beim axialen Spannen der Antriebsfeder 7 wird die Halterung 6 mit dem Reservoir 3 und dem Förderelement bei den Darstellungen nach unten bewegt und das Fluid 2 - genauer gesagt die nächste Dosis - aus dem Reservoir 3 über das Rückschlagventil 10 in die Druckkammer 11 des Druckerzeugers 5 gesaugt. Der Fluidraum 4 (Beutel) kollabiert in Abhängigkeit von der Entnahme von Fluid 2, wie beispielsweise und nur schematisch durch die gestrichelte Linie im unteren Bereich des Fluidraums 4 in Fig. 2 angedeutet.

Beim anschließenden Entspannen der Antriebsfeder 7 nach Betätigung des Sperrelements 8 zur Zerstäubung wird das Fluid 2 in der Druckkammer 11 unter Druck gesetzt, indem das Förderelement bei nun geschlossenem Rückschlagventil 10 vorzugsweise allein durch die Kraft der Antriebsfeder 7 wieder nach oben bewegt wird und nun als Druckstempel wirkt. Dieser Druck treibt das Fluid 2 durch die Austragsdüse 12 aus, wobei es in das vorzugsweise lungengängige Aerosol 14 zerstäubt wird, wie in Fig. 1 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Während des Zerstäubungsvorgangs bzw. -hubs wird das Reservoir 3 von der Antriebsfeder 7 wieder in seine Ausgangslage zurückbewegt. Das Reservoir 3 führt also vorzugsweise eine Hubbewegung während des Spannvorgangs und während des Zerstäubungsvorgangs aus.

Anstelle des Druckerzeugers 5 und/oder der Antriebsfeder 7 können auch sonstige Mittel und/oder Einrichtungen eingesetzt werden.

Der Zerstäuber 1 weist insbesondere ein erstes Gehäuseteil (Oberteil) 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares oder drehbares, zweites Gehäuseteil (Unterteil) 18 vorzugsweise mittels eines Sicherheitsverschlusses bzw. Halteelements 19 lösbar befestigt, insbesondere darauf aufgesteckt, ist. Insbesondere ist der Sicherheitsverschluß bzw. das Halteelement 19 derart ausgebildet, daß ein versehentliches Öffnen des Zerstäubers 1 bzw. Abziehen des zweiten Gehäuseteils 18 ausgeschlossen ist. Insbesondere muß zum Lösen des zweiten Gehäuseteils 18 das Halteelement 19 gegen Federkraft eingedrückt werden. Zum Einsetzen und/oder Auswechseln des Reservoirs 3 ist das zweite Gehäuseteil 18 vom Zerstäuber 1 lösbar. Das zweite Gehäuseteil 18 bildet vorzugsweise ein kappenartiges Gehäuseunterteil und/oder um- bzw. übergreift einen unteren freien Endbereich des Reservoirs 3.

Das zweite Gehäuseteil 18 kann relativ zum ersten Gehäuseteil 16 gedreht werden, wobei das Innenteil 17 mitgedreht wird. Dadurch wird die Antriebsfeder 7 über ein nicht im einzelnen dargestelltes, auf die Halterung 6 wirkendes Getriebe in axialer Richtung gespannt. Mit dem Spannen wird das Reservoir 3 axial nach unten bzw. mit seinem Endbereich (weiter) in das zweite Gehäuseteil 18 bzw. zu dessen stirnseitigem Ende hin bewegt, bis das Reservoir 3 eine in Fig. 2 angedeutete Endlage einnimmt. In diesem Zustand ist die Antriebsfeder 7 gespannt.

Der Zerstäuber 1 weist vorzugsweise eine Einrichtung zur zwangsweisen Belüftung des Reservoirs 3, insbesondere einer Außenhülle 23 des Reservoirs 3, auf.

Insbesondere kann beim erstmaligen Spannen ein beispielsweise bodenseitiges Anstechen bzw. Öffnen des Reservoirs 3 bzw. der Außenhülle 23. Beim Darstellungsbeispiel kommt eine axial wirkende, im Gehäuseteil 18 angeordnete Feder 20 am Reservoirboden 21 zur Anlage, die mit einem Anstechelement 22 eine bodenseitige, insbesondere gasdichte Versiegelung bei der erstmaligen Anlage zur Belüftung ansticht.

Die Einrichtung zur zwangsweisen Belüftung ist hier also durch das Anstechelement 22 gebildet, das von der Feder 20 gehalten oder gebildet ist. Jedoch sind auch andere konstruktive Lösungen möglich.

Es ist anzumerken, daß bei dem Anstechen zur Belüftung lediglich die Außenhülle 23 des Reservoirs 3 geöffnet wird. Der Fluidraum 4 (Beutel) mit dem Fluid 2 bzw. die Wandung 25 bleibt unbeschädigt.

Bei der Entnahme des Fluids 2 über das Förderelement kollabiert der flexible bzw. verformbare Beutel bzw. Fluidraum 4 bzw. wird die Wandung 25 verformt. Zum Druckausgleich kann die Umgebungsluft über die Belüftungs- bzw. Anstechöffnung in das Reservoir 3 bzw. die Außenhülle 23 nachströmen.

Die Einrichtung zur zwangsweisen Belüftung ist nur optional vorgesehen. Insbesondere kann die Einrichtung zur zwangsweisen Belüftung ganz entfallen, beispielsweise wenn die Außenhülle 23 des Reservoirs 3 ohnehin nicht zumindest im wesentlichen gasdicht ausgebildet ist und/oder wenn eine sonstige Einrichtung, wie ein Ventil oder eine bereits offene Belüftungsöffnung, zur Belüftung vorgesehen ist.

Zur Benutzung des Zerstäubers 1 muß zunächst das Reservoir 3 eingesetzt werden. Dies erfolgt vorzugsweise dadurch, daß das zweite Gehäuseteil 18 entfernt bzw. abgezogen wird. Anschließend wird das Reservoir 3 in das Innenteil 17 axial eingeführt bzw. eingeschoben. Hierbei erfolgt ein kopfseitiges Öffnen bzw. Anschließen. Dies erfolgt durch das Förderelement, also das Förderrohr 9, das eine vorzugsweise vorgesehene, insbesondere end- oder kopfseitige Versiegelung des Reservoirs 3 durchsticht und/oder anschließend durch einen insbesondere kopfseitigen Verschluß bzw. Anschluß 24, vorzugsweise mit einem Septum hindurch in das Innere des Reservoirs 3 bzw. Fluidraums 4 eingeführt wird. So wird die fluidische Verbindung zwischen dem Reservoir 3 - genauer gesagt zwischen dem Fluidraum 4 im Reservoir 3 - über das Förderrohr 9 zum Druckerzeuger 5 bzw. zur Druckkammer 11 hergestellt.

Anschließend wird das zweite Gehäuseteil 18 wieder aufgesetzt bzw. aufgeschoben. Nun kann das erstmalige Spannen des Zerstäubers 1 erfolgen. Vorzugsweise wird hierbei dann das Reservoir 3 durch das Anstechelement 22 bodenseitig angestochen, also zwangsweise belüftet, wir bereits erläutert.

Vor der erstmaligen Benutzung erfolgt nach dem Einsetzen bzw. fluidischen Anschließen des Reservoirs 3 ein vorzugsweise mehrmaliges Spannen und Auslösen des Zerstäubers 1. Durch dieses sogenannte Primen wird im Förderelement und/oder im Druckerzeuger 5 bis zur Austragsdüse 12 eventuell vorhandene Luft von dem Fluid 2 verdrängt. Anschließend ist der Zerstäuber 1 zur Ausgabe bzw. Inhalation bereit.

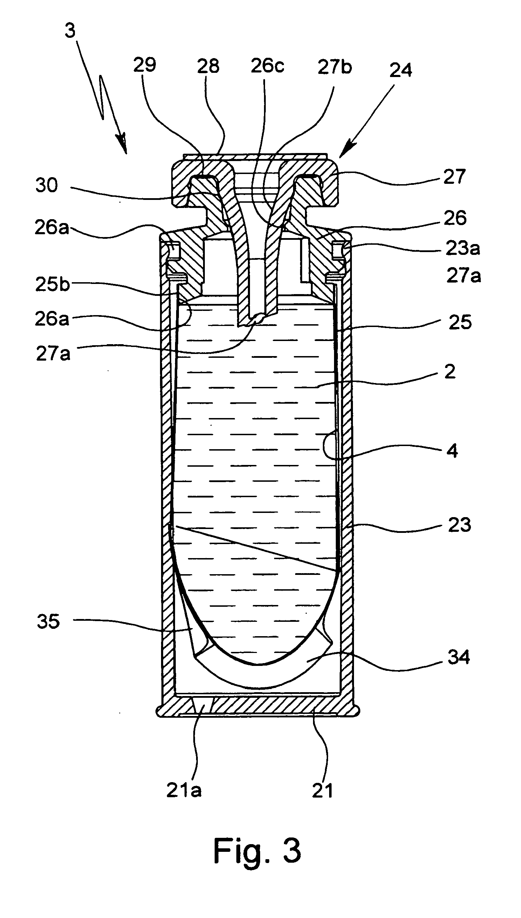

Fig. 3 zeigt in einem schematischen Schnitt einen besonders bevorzugten Aufbau des Reservoirs 3. Es ist anzumerken, daß die Darstellung nicht maßstabsgerecht ist.

Das Reservoir 3 bzw. dessen Verschluß 24 weist beim Darstellungsbeispiel ein erstes Verschlußteil 26 und ein zweites Verschlußteil 27 auf. Der Verschluß 24 besteht vorzugsweise nur aus den beiden Verschlußteilen 26 und 27 sowie der optionalen Versiegelung 28. Jedoch sind auch andere konstruktive Lösungen möglich.

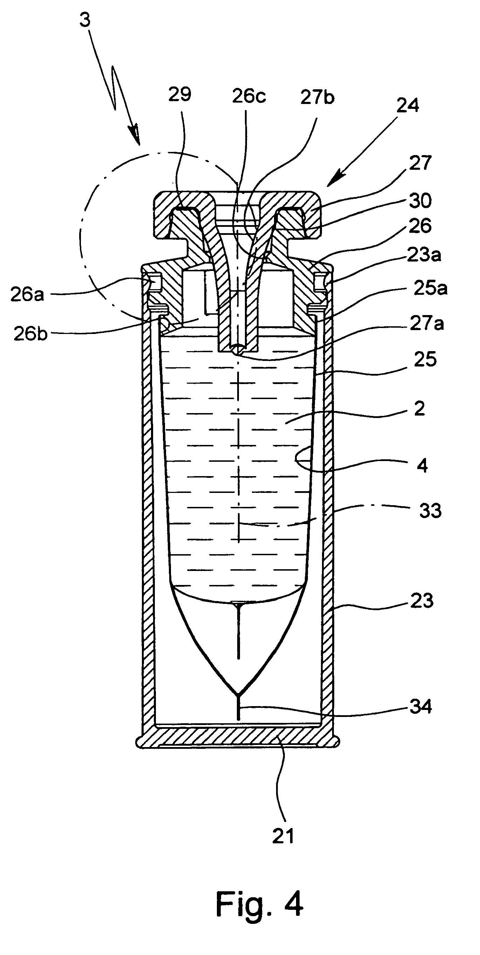

Fig. 4 zeigt das Reservoir 3 in einem zu Fig. 3 querverlaufenden Schnitt ohne optionale Versiegelung 28.

Beim Darstellungsbeispiel ist die Außenhülle 23 mit dem ersten Verschlußteil 26 verbunden, insbesondere verrastet. Vorzugsweise weist das erste Verschlußteil 26 hierzu eine Ausnehmung oder Ringnut 26a auf, in die Außenhülle 23 mit einem inneren bzw. radialen, vorzugsweise randseitigen Vorsprung 23a eingreift. Jedoch sind hier auch andere konstruktive Lösungen möglich.

Die Außenhülle 23 ist vorzugsweise zumindest im wesentlichen starr, länglich und/oder zylindrisch bzw. hohlzylindrisch, insbesondere hülsenartig, ausgebildet.

In Fig. 3 ist eine Belüftungsöffnung 21a im Behälterboden 21 gezeigt, die optional vorgesehen sein kann. Wenn die Belüftungdöffnung 21a vorgesehen ist, kann diese wahlweise zunächst verschlossen, beispielsweise versiegelt, sein und beim erstmaligen Benutzen des Zerstäubers 1 beispielsweise von dem Anstechelement 22 geöffnet werden, wie bereits oben beschrieben. Jedoch kann die Belüftungsöffnung 21a auch anfänglich offen sein. In diesem Fall kann die Öffnungseinrichtung bzw. die Feder 20 im Gehäuseunterteil mit dem Anstechelement 22 entfallen. Anstelle der Belüftungsöffnung 21a kann die Außenhülle 23 auch auf sonstige Weise zur Umgebung hin offen bzw. gasdurchlässig ausgebildet sein, um ein Kollabieren des Fluidraums 4 bzw. der Wandung 25 bei Entnahme von Fluid 2 aus dem Fluidraum 4 zu ermöglichen.

Der Fluidraum 4 bzw. die Wandung 25 wird vorzugsweise aus einem schlauchförmigen oder beutelförmigen Materialstück gebildet. Der Fluidraum 4 bzw. die Wandung 25 kann an dem dem Verschluß 24 gegenüberliegenden Ende wahlweise vor oder nach dem Anbringen des Verschlusses 24 bzw. ersten Verschlußteils 26 verschlossen werden.

Der Fluidraum 4 bzw. dessen Wandung 25 ist vorzugsweise ausschließlich mit dem ersten Verschlußteil 26 verbunden und/oder nur am verschlußseitigen Ende gehalten. Zur fluid- und insbesondere auch gasdichten Verbindung des ersten Verschlußteils 26 mit dem Fluidraum 4 bzw. der Wandung 25 weist das erste Verschlußteil 26 vorzugsweise einen Anschlußbereich 26b auf, der insbesondere ring- und/oder flanschartig ausgebildet und/oder durch eine äußere Umfangsfläche gebildet ist.

Die Verbindung des ersten Verschlußteils 26 mit der Wandung 25 bzw. dem Beutel / Fluidraum 4 erfolgt vorzugsweise vor der Befüllung mit Fluid 2.

Der Fluidraum 4 bzw. die Wandung 25 ist vorzugsweise in einem End- oder Ringbereich 25a mit dem ersten Verschlußteil 26 bzw. dessen Anschlußbereich 26b fluiddicht und insbesondere gasdicht verbunden. Besonders bevorzugt erfolgt diese Verbindung durch Warmformung bzw. Warmverformung des ersten Verschlußteils 26 und/oder in der Art einer Siegelung, insbesondere durch Einwirkung von Hitze und Druck, und/oder auf jede sonstige geeignete Art und Weise, beispielsweise durch Kleben, Verformen, Verpressen und/oder Verschweißen. Beim Darstellungsbeispiel wird das erste Verschlußteil 26 vorzugsweise als Vorformling in ein offenes Ende bzw. den Ringbereich 25a des insbesondere noch schlauchförmigen oder hülsenartigen Fluidraums 4 bzw. der Wandung 25 eingeführt und dann durch Warm(ver)formen mit der Wandung 25 - hier der Innenseite der Wandung 25 im Ringbereich 25a - fest verbunden.

Vorzugsweise wird das Fluid 2 in den Fluidraum 4 durch eine Einfüllöffnung 26c des ersten Verschlußteils 26 eingefüllt. Anschließend wird der Verschluß 24 durch Aufsetzen des zweiten Verschlußteils 27 und Verbinden mit dem ersten Verschlußteil 26 verschlossen. Grundsätzlich ist es aber auch möglich, das Fluid 2 von dem anderen Ende des Fluidraums 4 her erst nach Schließen des Verschlusses 24 einzufüllen und dann erst dieses Ende des Fluidraums 4 bzw. der Wandung 25 zu schließen.

Das zweite Verschlußteil 27 weist vorzugsweise ein Septum 27a und/oder eine Einführöffnung 27b für ein Förderelement, wie das Förderrohr 9, des Zerstäubers 1 zur Entnahme von Fluid 2 aus dem Fluidraum 4 auf.

Zum Verschließen des Verschlusses 24 wird das zweite Verschlußteil 27 vorzugsweise in die Einfüllöffnung 26c des ersten Verschlußteils 26 - insbesondere mit dem Septum 27a voraus - eingeführt bzw. eingesetzt und/oder auf das erste Verschlußteil 26 aufgesetzt.

Die beiden Verschlußteile 26, 27 werden dann fluiddicht und insbesondere gasdicht miteinander in einem Verbindungsbereich 29 verbunden. Insbesondere werden die beiden Verschlußteile 26, 27 in dem Verbindungsbereich 29 verschweißt, besonders bevorzugt durch Ultraschallschweißen. Jedoch ist es grundsätzlich auch möglich, die beiden Verschlußteile 26, 27 auf sonstige Weise in dem Verbindungsbereich 29 zum Schließen des Verschlusses 24 und insbesondere zum hermetischen Verschließen des Fluidraums 4 im Verbindungsbereich 29 miteinander zu verbinden.

Der Verbindungsbereich 29 ist vorzugsweise umlaufend oder ringförmig ausgebildet. Insbesondere umgibt der Verbindungsbereich 29 die Einfüllöffnung 26c und/oder Einführöffnung 27b konzentrisch.

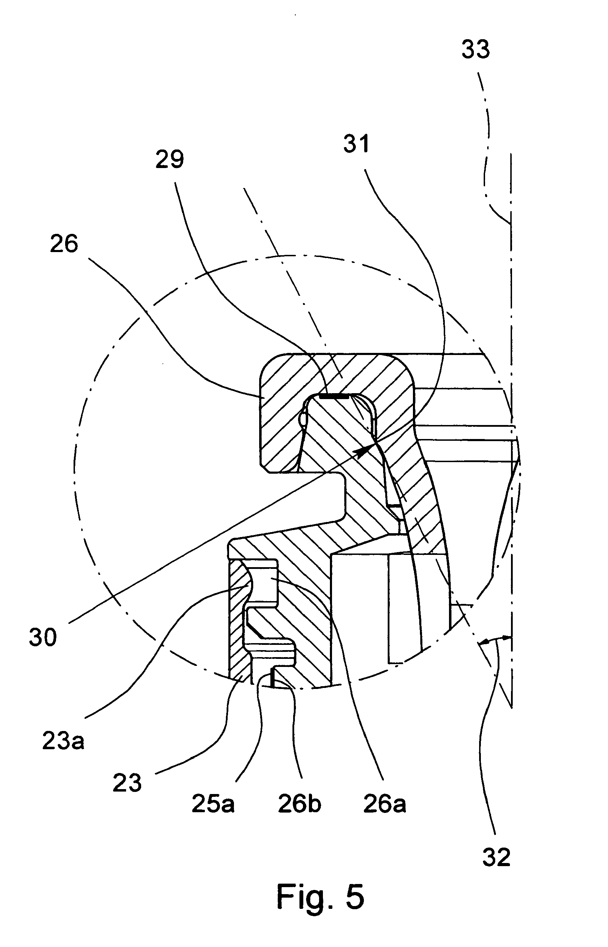

Besonders bevorzugt ist der Verbindungsbereich 29 zwischen zwei sich im wesentlichen radial erstreckenden und axial aufeinanderliegenden Flanschabschnitten der beiden Verschlußteile 26, 27 gebildet, wie insbesondere in Fig. 5 angedeutet, die einen vergrößerten Ausschnitt von Fig. 4 zeigt. Jedoch sind hier auch andere konstruktive Lösungen bzw. Anordnungen oder Konfigurationen des Verbindungsbereichs 29 möglich.

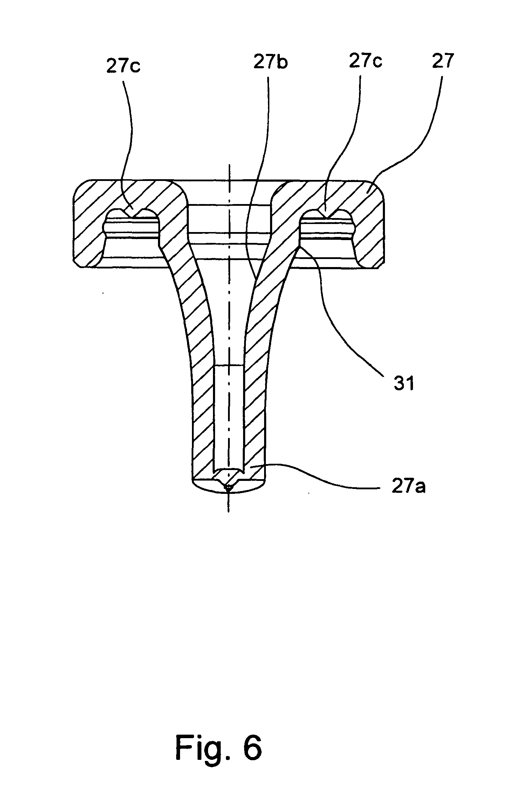

Fig. 6 zeigt in einem vergrößerten Schnitt das zweite Verbindungsteil 27 vor dem Einbau, also separat. In Fig. 6 ist zu erkennen, daß das zweite Verschlußteil 27 vorzugsweise einen Ringwulst 27c o. dgl. - hier mit einem sich verjüngenden und/oder insbesondere im wesentlichen dreieckigen Querschnitt - aufweist, um das Material zum Verschweißen der beiden Verschlußteile 26, 27 bereitzustellen.

Die beiden Verschlußteile 26, 27 sind vorzugsweise aus einem geeigneten und/oder dem gleichen Kunststoff, insbesondere Polyethylen, oder aus einem sonstigen geeignetem Material hergestellt. Das zum Verbinden der beiden Verschlußteile 26 und 27 vorzugsweise angeschmolzene oder geschmolzene Material kann durch den optionalen Ringwulst 27c, wie bereits erwähnt, oder auf sonstige Weise bereitgestellt oder während des Verbindungsprozesses zugegeben werden. Beispielsweise kann das Material einen Ring bilden. Das Material kann aufgepudert oder aufgeflockt sein oder werden. Das Material kann dem Material des ersten und/oder zweiten Verschlußteils 26, 27 entsprechen oder Bestandteile davon enthalten. Es kann sich bei dem Verbindungsmaterial jedoch auch um ein sonstiges Material oder um ein Material mit einer anderen Zusammensetzung handeln. Je nach Verfahren kann das Material auch eine andere Schmelztemperatur als das Material des ersten und/oder zweiten Verschlußteils 26, 27 aufweisen.

Wie bereits erwähnt, erfolgt das Verbinden der beiden Verschlußteile 26, 27 vorzugsweise durch Verschweißen, insbesondere mittels Ultraschall oder durch Laserschweißen. Hierbei wird dann das vorzugsweise durch den optionalen Ringwulst 27c bereit gestellte Material und/oder sonstiges Material angeschmolzen oder geschmolzen und verbindet die beiden Verschlußteile 26, 27 im Verbindungsbereich 29, wie in den Fig. 3 bis 5 schematisch angedeutet.

Das Reservoir 3 bzw. der Verschluß 24 weist eine Sperre 30 zum Verhindern des Eindringens von beim Verschließen des Verschlusses 24 bzw. Verbinden der Verschlußteile 26, 27 potentiell entstehenden Partikeln, wie Schweißpartikel bzw. Austrieb, oder sonstigen Verschmutzungen in das Innere des Reservoirs 3 bzw. in das Fluid 2 und/oder in den Fluidraum 4 auf. Primär dient die Sperre 30 dem Verhindern eines Eindringens von Partikeln, so daß nachfolgend auch von Partikelsperre gesprochen wird, auch wenn diese zusätzlich oder alternativ das Eindringen sonstiger Verschmutzungen verhindern oder zumindest minimieren kann.

Die Sperre 30 ist wie beansprucht durch einen Paßsitz mit Auflagebereich 31 der beiden Verschlußteile 26, 27 gebildet, wie in der Vergrößerung gemäß Fig. 5 angedeutet.

Vorzugsweise wird zwischen den beiden Verschlußteilen 26, 27 ein vorzugsweise zumindest im wesentlichen radialer Paß-, Klemm- oder Preßsitz gebildet, der die Sperre 30 bildet.

Die Sperre 30 ist vorzugsweise ringförmig ausgebildet. Die Sperre 30 ist vorzugsweise durch stumpfe Geometrien gebildet, die zur Auflage kommen; beim Darstellungsbeispiel sind nur stumpfe Winkel oder glatte oder gerundete Oberflächen vorgesehen, die zur Anlage oder Auflage bei der Bildung der Sperre 30 kommen, insbesondere um ein unerwünschtes Verschweißen im Bereich der Sperre 30 zu verhindern. Besonders bevorzugt sind die Verschlußteile 26, 27 im Bereich der Sperre 30 nicht fest verbunden bzw. verschweißt.

Die Verbindung der beiden Verschlußteile 26, 27 im Verbindungsbereich 29 erfolgt beim Darstellungsbeispiel im wesentlichen axial, also quer und insbesondere zumindest im wesentlichen senkrecht zu der Passung bzw. Klemmung der Sperre 30. Dies gestattet es, daß während des Verbindens, insbesondere Verschweißens, der beiden Verschlußteile 26, 27 und der damit dabei einhergehenden Bewegung in Verbindungsrichtung (beim Darstellungsbeispiel zumindest im wesentlichen senkrecht zur Flächenerstreckung des Verbindungsbereichs 29 bzw. zumindest im wesentlichen in Richtung der in Fig. 4 und 5 angedeuteten Achse 33 des Behälters 3 bzw. Verschlusses 24 oder der Einführrichtung des Förderelements) die Sperre 30 bereits wirksam wird, die beiden Verschlußteile 26, 27 insbesondere also zumindest im wesentlichen über den gesamten Schweißweg die Sperre 30 bilden.

Beim Darstellungsbeispiel wirken die Ultraschallschwingungen zum Verschweißen vorzugsweise zumindest im wesentlichen axial bzw. in Längserstreckung des Reservoirs 3. Die Reibung nach unten bzw. in axialer Richtung bzw. im Verbindungsbereich 29 bewirkt dann das Verschweißen. Wenn die beiden Verschlußteile 26 und 27 nicht durch Ultraschall verschweißt, sondern auf andere Weise, insbesondere durch Laserschweißen, mit einander verbunden werden, kann die Sperre 30 bzw. der die Sperre 30 bildende Bereich, insbesondere des ersten und/oder zweiten Verschlußteils 26, 27, auch eine andere Geometrie aufweisen und beispielsweise durch eine trichterförmige oder lippenartige Geometrie und/oder radiale Dichtungsgeometrie, besonders bevorzugt in der Art einer Wellendichtung, gebildet sein.

Generell verläuft die Verbindungsrichtung der beiden Verschlußteile 26, 27 also quer bzw. senkrecht zu der Sperre 30 bewirken Klemmung bzw. Klemmkraft bzw. Passung, vorzugsweise zwischen den beiden Verschlußteilen 26, 27.

Bei dem der beanspruchten Losung entsprechenden Dartellungsbeispiel sind die Verschlußteile 26, 27 zur Bildung der Sperre 30 bzw. im Auflagebereich 31 konisch ineinander geschoben. Besonders bevorzugt verläuft der Auflagebereich 31 oder zumindest ein von dem ersten oder zweiten Verschlußteil 26 bzw. 27 gebildeter Auflageabschnitt leicht konisch, insbesondere mit einem Winkel 32 geneigt zu einer Achse 33, wie in Fig. 5 schematisch angedeutet.

Die Achse 33 verläuft hier zumindest im wesentlichen in Richtung der Längsachse des Reservoirs 3, in einer Zusammenbaurichtung der beiden Verschlußteile 26, 27, senkrecht zur flächigen Erstreckung des Verbindungsbereichs 29, axial bezüglich des Verschlusses 24 und/oder in der bevorzugten Einführrichtung des Förderelements in das Reservoir 3.

Der Winkel 32 entspricht vorzugsweise im wesentlichen dem Verhältnis von Schweißweg zu dem erforderlichem Spaltmaß zwischen dem zweiten Verschlußteil 27 und dem ersten Verschlußteil 26 im Bereich der Sperre 30 bei aufgesetztem zweiten Verschlußteil 27 vor dem Verschweißen.

Der Winkel 32 beträgt vorzugsweise weniger als 30°, insbesondere weniger als 20°, besonders bevorzugt etwa 15° oder weniger.

Die Bezeichnungen "radial" und "axial" beziehen sich bei der vorliegenden Erfindung generell vorzugsweise auf die bereits genannte Richtung bzw. Achse 33.

Wie bereits erwähnt, ist die Sperre 30 fluidseitig und separat bzw. getrennt vom Verbindungsbereich 29 angeordnet bzw. derart ausgebildet, daß die Sperre 30 den Fluidraum 4 gegen das Eindringen von Partikeln, wie Schweißpartikeln (nicht dargestellt), oder sonstigen Verschmutzungen, die beim Verbinden der beiden Verschlußteile 26, 27 insbesondere durch Verschweißen auftreten können, (ausreichend) abschließt.

Die Sperre 30 ist vom Verbindungsbereich 29 vorzugsweise ausreichend beabstandet, um zu verhindern, daß die beiden Verschlußteile 26, 27 im Bereich der Sperre 30 ebenfalls verschweißt werden, wenn die beiden Verschlußteile 26, 27 im Verbindungsbereich 29 (insbesondere durch Ultraschallschweißen) verschweißt werden.

Gemäß Anspruch 1 ist die Sperre 30 vom Verbindungsbereich 29 axial und radial beabstandet.

Die Sperre 30 ist somit näher zum Fluidraum 4 als der Verbindungsbereich 29 angeordnet.

Die Sperre 30 ist vorzugsweise umlaufend oder ringförmig ausgebildet, und insbesondere konzentrisch zum Verbindungsbereich 29 angeordnet.

Die Sperre 30 ist vorzugsweise derart ausgebildet, daß sich der Anlagebereich 31 vom freien Ende des Verschlusses 24 ausgehend zum Inneren des Fluidraums 4 hin verjüngt. Jedoch sind hier auch andere konstruktive Lösungen möglich.

Die Wandung 25 ist zumindest im wesentlichen oder teilweise verformbar bzw. flexibel ausgebildet, um ein möglicht leichtes Kollabieren des Fluidraums 4 bei der Entnahme von Fluid 2 zu gestatten. Der Fluidraum 4 ist nämlich zumindest weitestgehend gasdicht abgeschlossen. Die Wandung 25 ist dementsprechend weitestgehend gasdicht ausgebildet.

Die Wandung 25 ist vorzugsweise mehrschichtig aufgebaut, insbesondere weist die Wandung 25, eine vorzugsweise metallische Sperrschicht (insbesondere eine Metallfolie) und eine Innenschicht sowie gegebenenfalls weitere Schichten auf. Die einzelnen Schichten können beispielsweise durch Beschichten, Auflaminieren oder auf sonstige geeignete Art und Weise gebildet werden. Die Sperrschicht ist insbesondere als Aluminiumschicht oder -folie ausgeführt.

Besonders bevorzugt weist die Wandung 25 bzw. das verwendete Folienmaterial eine äußere Schutzschicht, vorzugsweise aus PET, eine vorzugsweise metallische Zwischen- oder Sperrschicht, insbesondere aus Aluminium, und eine Innenbeschichtung, insbesondere aus PE, auf.

Die Wandung 25 bzw. deren Schichtaufbau ist vorzugsweise folienartig ausgebildet bzw. aus Folienmaterial hergestellt.

Bei der Herstellung wird aus der Wandung 25 bzw. dem die Wandung 25 bildenden Folienmaterial zunächst eine Hülse bzw. hohlzylindrische Form bzw. ein Schlauch - insbesondere durch Längsverschweißen eines entsprechenden Materialstreifens - gebildet.

Nach einem eventuell erforderlichen Ablängen bei Endlosherstellung werden die hülsenartigen Stücke verschlossen, um den jeweiligen Fluidraum 4 zu bilden.

Der Verschluß 24 verschließt das Reservoir 3 bzw. den Fluidraum 4 an einem ersten Ende, insbesondere in einem Kopfbereich bzw. stirnseitig.

Vorzugsweise wird zunächst der Verschluß 24 bzw. das Verschlußteil 26 angebracht, besonders bevorzugt angespritzt oder angeformt. Hierzu wird vorzugsweise ein geeignetes Material, wie ein PE (Polyethylen) oder ein PET (Polyethylentherephtalat), das sich mit der Wandung 25 verbindet, für das Verschlußteil 26 gewählt. Beispielsweise kann sich das Verschlußteil 26 direkt mit der Innenseite der Wandung 25 verbinden.

Vorzugsweise ist die Wandung 25 nur mit dem Verschlußteil 26 fest oder unlösbar verbunden.

Der Fluidraum 4 bzw. die Wandung 25 ist insbesondere in einem dem Verschluß 24 gegenüberliegenden Endbereich verschlossen, insbesondere durch Verschweißen, Versiegeln o. dgl. der Wandung 25, wodurch eine Quernaht 34 gebildet wird, wie in Fig. 3 und 4 angedeutet. Dieses endseitige Schließen des Fluidraums 4 kann wahlweise vor oder nach dem Anbringen des Verschlusses 24 bzw. Verschlußteils 26 erfolgen.

Das Verschließen des Fluidraums 4 am freien Ende erfolgt vorzugsweise nach dem Anbringen des Verschlußteils 26 bzw. dem Anbringen des Verschlusses 24. Jedoch kann das Verschließen des Reservoirs 3 bzw. Fluidraums 4 an dem dem Verschluß 24 gegenüberliegenden Ende - auch zuerst, also vor dem Anbringen des Verschlußteils 26 erfolgen.

Das Füllen des Reservoirs 3 bzw. des Fluidraums 4 mit dem Fluid 2 kann wahlweise entweder über den Verschluß 24 - also das eigentliche Entnahmeende - oder über das andere, hier bodenseitige bzw. freie Ende erfolgen. Im ersten Fall wird das Fluid 2 vor dem Verschließen des Verschlusses 4, insbesondere vor dem Einsetzen des zweiten Verschlußteils 27 mit dem Fluid 2 gefüllt (das Verschlußteil 27 wird vorzugsweise gasdicht mit dem Verschlußteil 26 verschweißt, um den Verschluß 24 zu verschließen).

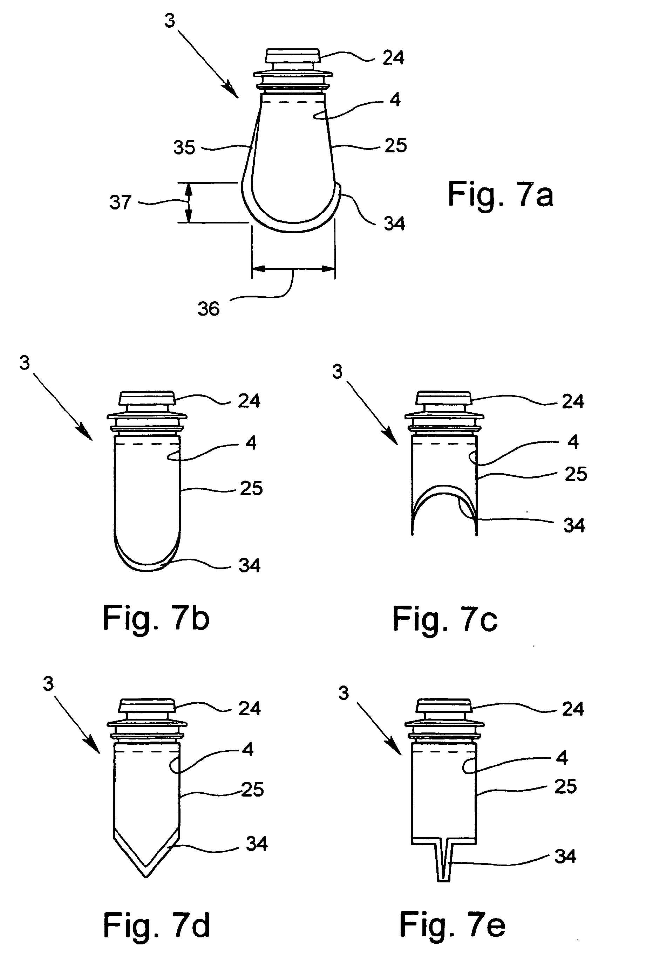

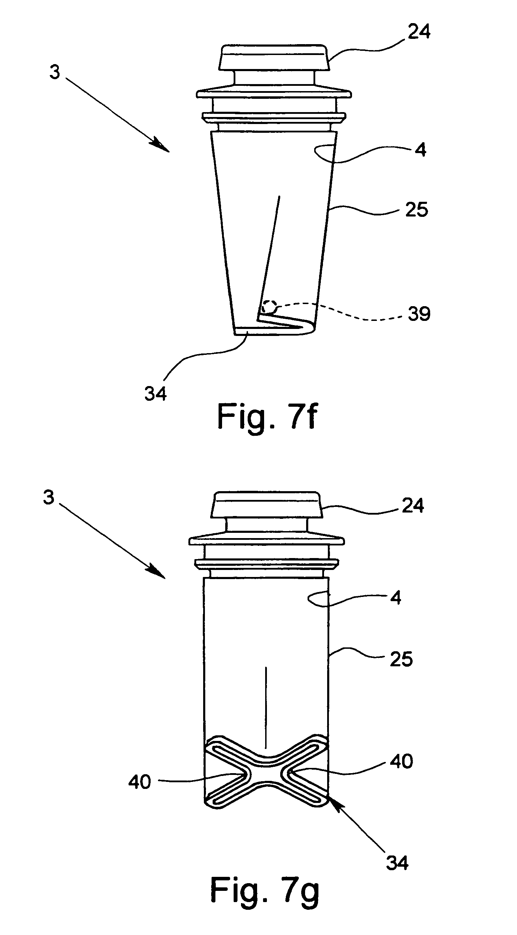

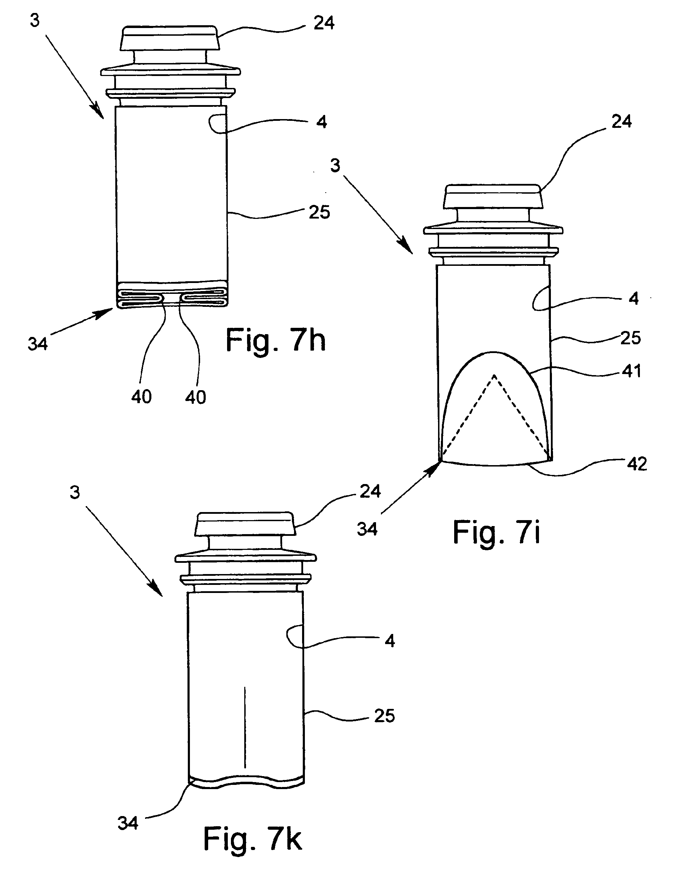

Nachfolgend werden verschiedene bevorzugte Formen des Fluidraums 4 bzw. der Wandung 25 anhand der nur schematischen Darstellungen gemäß Fig. 7 erläutert. Diese Darstellungen zeigen jeweils das Reservoir 3 ohne Außenhülle 23.

Fig. 7a zeigt eine besonders bevorzugte Form des Fluidraums 4 bzw. der Wandung 25. Zusätzlich zu der Quernaht 34 ist hier eine optionale Längsnaht 35 des Fluidraums 4 bzw. der Wandung 25 angedeutet. Bei dieser beutelförmigen Ausgestaltung ist die größte Breite 36 im Bereich des dem Verschluß 24 abgewandten Endes des Fluidraums 4 bzw. im Bereich der Quernaht 34 vorzugsweise größer als der Durchmesser des Verschlusses 24 bzw. der Wandung 25 zum Verschluß 24 hin. Die Ausführungsvariante gemäß Fig. 7a gestattet insbesondere ein optimiertes bzw. sehr hohes Füllvolumen.

Der Fluidraum 4 weist vorzugsweise ein gerundetes und/oder flaches Ende im Bereich der Quernaht 34 auf. Jedoch kann die Quernaht 34 beispielsweise auch nur quer bzw. gerade, ggf. auch schräg verlaufen.

Vorzugsweise geht die Quernaht 34 in die Längsnaht 35 über.

Bei der vorliegenden Ausgestaltung ist der Abstand 37 des Bereichs des größten Durchmessers 36 bzw. der größten Bereite 36 zum freien Ende des Fluidraums 4 hin vorzugsweise kleiner als der Durchmesser des Verschlusses 24 oder des Fluidraums 4 im Bereich des Verschlusses 24.

Beim Zusammenbau des Reservoirs 3 bzw. Einbau des Beutels bzw. Fluidraums 4 in die Außenhülle 23 wird die Wandung 25 derart radial zusammengedrückt oder gefaltet, daß die Breite 36 derart reduziert wird, daß die Wandung 25 in die Außenhülle 23 paßt, wie in dem Schnitt gemäß Fig. 3 schematisch angedeutet. Hierbei wird die optionale Längsnaht 35 vorzugsweise umgelegt bzw. umgefaltet.

Aus dem anderen Längsschnitt gemäß Fig. 4 ist ersichtlich, daß der Fluidraum 4 bzw. die Wandung 25 mit ihrer Breite bzw. Dicke ausgehend vom Verschluß 24 zum anderen Ende hin vorzugsweise abnimmt bzw. flacher wird, besonders bevorzugt ein verhältnismäßig flaches freies Ende bildet. Bei der Entnahme von Fluid 2 aus dem Fluidraum 4 kollabiert der Fluidraum 4 insbesondere in dieser Querrichtung weiter. Jedoch sind auch andere Verformungen möglich.

Bei der Ausführungsvariante gemäß Fig. 7b weist der Fluidraum 4 bzw. die Wandung 25 einen zumindest im wesentlichen konstanten Durchmesser über die gesamte axiale Erstreckung auf.

Bei der Ausführungsvariante gemäß Fig. 7b verläuft die Quernaht 34 vorzugsweise gerundeter bzw. mit einem kleineren Krümmungsradius als dies bei der Ausführungsvariante gemäß Fig. 7a der Fall ist. Dies kann einem leichteren Kollabieren zuträglich sein und/oder erleichtert die Kollabierung.

Bei der Ausführungsvariante gemäß Fig. 7c weist der Fluidraum 4 bzw. die Wandung 25 eine vorzugsweise konvex verlaufende Quernaht 34 auf. Dies kann insbesondere Spannungen im Bereich der Quernaht 34 reduzieren.

Bei der Ausführungsvariante gemäß Fig. 7d weist der Fluidraum 4 bzw. die Wandung 25 eine vorzugsweise zumindest im wesentlichen V-förmig und/oder geneigt verlaufende Quernaht 34 auf. Wie bereits erwähnt, kann die Quernaht 34 auch zumindest im wesentlichen nur gerade verlaufen (wahlweise entweder im wesentlichen senkrecht oder schräg bzw. geneigt zur Längsachse 33 des Reservoirs 3 bzw. Einführrichtung, die hier nicht dargestellt ist).

Bei der Ausführungsvariante gemäß Fig. 7e ist die Quernaht 34 zumindest im wesentlichen T-förmig ausgebildet. Dies vereinfacht die Gestaltung von Versiegelungsbacken (nicht dargestellt) zur Bildung der Quernaht 34 und/oder die Fertigung.

Bei der Ausführungsvariante gemäß Fig. 7f wird der Fluidraum 4 bzw. die Wandung 25 nach dem Verschließen gefaltet, hier besonders bevorzugt längs gefaltet, zumindest im Bereich des freien Endes bzw. der Quernaht 34. Besonders bevorzugt ist die Faltung derart ausgeführt, daß der Fluidraum 4 zum freien Ende hin eine sich verjüngende und/oder zumindest im wesentlichen nicht zunehmende Außenkontur aufweist, um das Aufschieben der Außenhülle 32 zu erleichtern oder ermöglichen.

Bei der dargestellten Ausführungsvariante weist der Fluidraum 4 bzw. die Wandung 25 eine sogar zumindest im wesentlichen gerade und/oder quer zur Längserstreckung verlaufende Quernaht 34 auf. Jedoch sind hier auch andere Konfigurationen bzw. Quernahtformen möglich.

Um den Beutel bzw. Fluidraum 4 im gefalteten Zustand zumindest temporär für den Zusammenbau bzw. für das Aufschieben der Außenhülse 23 zu halten bzw. zu fixieren, ist beim Darstellungsbeispiel vorzugsweise ein Befestigungs- oder Verbindungsmittel vorgesehen, das hier besonders bevorzugt durch partielles außenseitiges Zusammenkleben realisiert wird, wie durch den vorzugsweise punktförmigen Klebebereich 39 in Fig. 7f schematisch angedeutet. Zum Zusammenkleben wird insbesondere ein Heißkleber verwendet. Beispielsweise kann aber auch ein Streifen oder ein sonstiges äußeres Fixiermittel, beispielsweise ein aufgeschobener Ring, oder eine sonstige Einführhilfe verwendet oder eingesetzt werden, um den Beutel bzw. Fluidraum 4 zumindest temporär für den Einbau in die Außenhülle 23 in eine gewünschte Form zu bringen oder darin zu halten oder zumindest in der äußerlichen Ausdehnung zu begrenzen. Eine derartige Einführ- oder Montagehilfe kann jedoch auch zusätzlich oder alternativ durch ein (nicht dargestelltes) separates Werkzeug, beispielsweise ein Einführtrichter, erreicht werden, das beispielsweise beim Einführen des Beutels bzw. des Fluidraums 4 in die Außenhülle 32 eine entsprechende Formung und/oder Führung des Beutels bzw. Fluidraums 4 bewirkt.

Ein besonderer Vorteil der Ausführungsvariante gemäß Fig. 7f liegt darin, daß eine hohe Formstabilität des Fluidraums 4 bzw. der Wandung 25 zum Einführen in die Außenhülle 23 erreichbar ist.

Bei der Ausführungsvariante gemäß Fig. 7g verläuft die Quernaht 34 vorzugsweise räumlich. Insbesondere erfolgt bei der Herstellung eine räumliche Formgebung durch die Art des Verlaufs der Quernaht 34 bzw. der durch die Quernaht 34 miteinander verbundenen Bereiche der Wandung 25.

Bei der Ausführungsvariante gemäß Fig. 7g wird eine in der Stirnansicht vorzugsweise zumindest im wesentlichen X-förmige Quernaht 34 gebildet.

Ein besonderer Vorteil liegt darin, daß eine hohe Formstabilität des Fluidraums 4 bzw. der Wandung 25 zum Einführen in die Außenhülle 23 erreichbar ist.

Bei der Ausführungsvariante gemäß Fig. 7h ist der Fluidraum 4 bzw. die Wandung 25 zumindest im Bereich zum freien Ende hin bzw. zur Quernaht 34 hin längsseitig eingefaltet, insbesondere auf gegenüberliegenden Seiten, wie schematisch durch die Einbiegungen oder Einbuchtungen oder Einfaltungen 40 angedeutet. Besonders bevorzugt wird dies durch eine entsprechende Formgebung der Quernaht 34 bzw. räumliche Quernaht 34 bzw. entsprechendes endseitiges Verschweißen oder Versiegeln des im Fluidraum 4 bzw. die Wandung 25 bildenden Materialstücks / Folienmaterials erreicht. Auch so kann eine relativ hohe Formstabilität des Fluidraums 4 bzw. Wandung 25 zum Einführen in die Außenhülle 23 erreicht werden.

Bei den voranstehend beschriebenen Ausführungsvarianten kann das Schweißen bzw. Versiegeln oder sonstige Schließen des Beutels bzw. Fluidraums 4 besonders bevorzugt in einer Ebene erfolgen. Nachfolgend werden andere räumliche Quernahtformen beschrieben, bei denen das Schweißen, Versiegeln oder auf sonstige Weise erfolgende Verbinden nicht in einer Ebene, sondern in einer dreidimensionalen Raumform erfolgt. Besondere Vorteile liegen darin, daß eine sehr hohe Formstabilität des Fluidraums 4 bzw. der Wandung 25 insbesondere zum freien Ende hin bzw. im Bereich der Quernaht 34 erreichbar ist und/oder daß der Durchmesser des Fluidraums 4 bzw. der Wandung 25 zum freien Ende hin bzw. im Bereich der Quernaht 34 sehr definiert vorgegeben bzw. beeinflußt werden kann, insbesondere sich zum freien Ende hin nicht ändert oder bedarfsweise abnimmt. Weiter kann die Form des freien Endes insbesondere im Hinblick auf ein leichtes Einführen in die Außenhülle 23 optimiert werden beispielsweise durch eine sich verjüngende Form.

Bei der Ausführungsvariante gemäß Fig. 7i wird eine Seite der Wandung 25 bzw. des Materialstücks am freien Ende soweit eingedrückt, bis sie auf der anderen Seite aufliegt. Hierbei kommen die oberen Kanten der beiden Seiten nicht aufeinanderzuliegen, sondern liegt die Kante der eingedrückten Seite etwas zurück versetzt innen auf der nicht eingedrückten Seite. Die so entstehende Quernaht 34 ist nur schematisch angedeutet. Sie setzt sich beispielsweise aus einem im wesentlichen parabelförmigen Teil 41 und einem im wesentlichen kreisbogenförmigen Teil 42 zusammen.

Jedoch sind auch andere räumliche Konfigurationen möglich. Beispielsweise kann das Eindrücken der einen Seite gegen die anderen Seiten am Ende des Fluidraums 4 bzw. der Wandung 25 derart erfolgen, daß keine runde, sondern eine zumindest im wesentlichen eckige, besonders bevorzugt V-förmige oder dreieckige Form oder Außenkontur erreicht wird, wie gestrichelt in Fig. 7i angedeutet. Besonders bevorzugt ist der Beutel bzw. Fluidraum 4 oder die Quernaht 34 in der Stirnansicht dann im wesentlichen V-förmig, wobei der eingeschlossene Winkel dann besonders bevorzugt weniger als 75 ° beträgt, so daß der Beutel bzw. Fluidraum 4 auch direkt in die Außenhülle 23 paßt.

Fig. 7k zeigt eine weitere Ausführungsvariante mit einem gewellten Beutel- bzw. Fluidraumende bzw. mit einer räumlich gewellten Quernaht 34. Auch hier kann durch die Formgebung der Quernaht 34 wieder erreicht werden, daß der Beutel bzw. Fluidraum 4 direkt in die Außenhülle 23 paßt.

Generell ist anzumerken, daß der Fluidraum 4 bzw. die Wandung 25 eine zumindest im wesentlichen längliche, vorzugsweise sich zum freien Ende hin verjüngende und/oder zylindrische Form aufweist, was die Montage, insbesondere das Aufschieben der Außenhülle 23 bzw. Einführen in die Außenhüllse 23, erleichtert. Jedoch sind auch andere konstruktive Lösungen möglich.

Weiter ist generell anzumerken, daß ein definiertes Falten des Beutels bzw. Fluidraums 4 und/oder die räumlich definierte verlaufende Quernaht 34 eine unerwünschte oder undefinierte Knickbildung des Beutels bzw. Fluidraums bei der Befüllung verhindern kann. Dies ist insbesondere dann wichtig, wenn eine Befüllung zumindest im wesentlichen ohne Druck erfolgt und/oder wenn ein definiertes Expandieren vor der Befüllung, beispielsweise durch Aufblasen, sichergestellt sein soll.

Wie bereits erwähnt, kann der Beutel bzw. Fluidraum 4 auch nach der Herstellung der Quernaht 34 generell gefaltet werden. Dies kann wahlweise vor oder nach der Befüllung mit Fluid 2 und/oder einem Expandieren durch Aufblasen erfolgen. So kann insbesondere erreicht werden, daß der Beutel bzw. Fluidraum 4 eine Form annimmt, die ein Einführen in die Außenhülle 23 ermöglicht oder zumindest erleichtert.

Alternativ oder zusätzlich kann ein nicht dargestelltes Einführwerkzeug, wie ein Einführtrichter o. dgl., verwendet werden, um den Zusammenbau - insbesondere das Aufschieben der Außenhülle 23 auf den Beutel bzw. Fluidraum 4 - zu ermöglichen oder zu erleichtern.

Alternativ oder zusätzlich kann die Außenhülle 23 bedarfsweise auch temporär verformt, beispielsweise an ihrem Aufnahmeende etwas flachgedrückt werden, um den Beutel bzw. Fluidraum 4 besser oder leichter aufnehmen zu können. Dies würde insbesondere das Einführen des biberschwanzartig ausgebildeten bzw. zum freien Ende hin verbreiterten Beutels bzw. Fluidraums 4 (beispielsweise gemäß Fig. 7a) erleichtern oder ermöglichen.

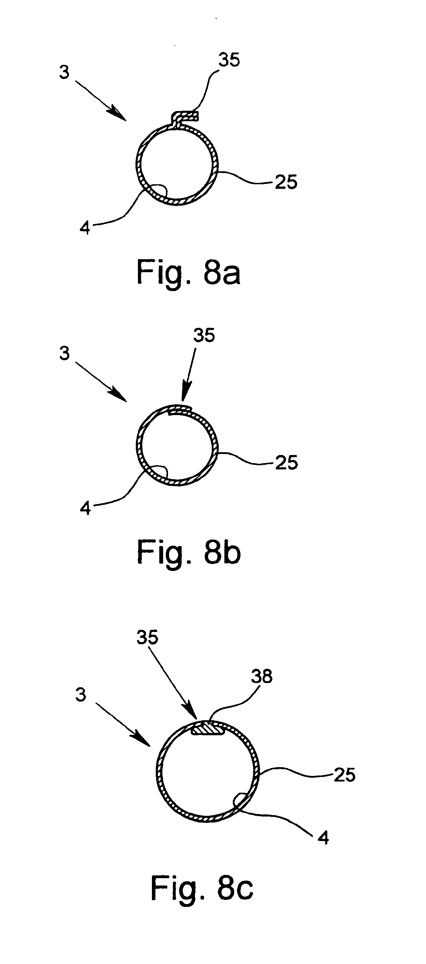

Wie bereits erwähnt, wird der Fiuidraum 4 bzw. die Wandung 25 vorzugsweise aus einem länglichen Materialstreifen hergestellt, der zur Bildung des vorzugsweise beutelförmigen bzw. schlauchförmigen oder hülsenartigen Fluidraums 4 längsseitig verschlossen, insbesondere längsverschweißt, wird. Fig. 8 zeigt Darstellungen von zwei möglichen Ausführungsvarianten zur Längsverschweißung. Der Fluidraum 4 bzw. die Wandung 25 ist jeweils in einen nur schematischen, nicht maßstabsgerechten Querschnitt ohne Außenhülle 23 und ohne Quernaht 34 dargestellt.

Bei der Ausführungsvariante gemäß Fig. 8a ist das die Wandung 25 bildende Materialstück durch die eigentlich radial abstehende Längsnaht 35 längsseitig verbunden. Die Längsnaht 35 ist vorzugsweise umgeklappt oder umgefaltet, wie in Fig. 8a nur schematisch angedeutet. Das Verbinden der von Rändern des Materialstücks im Bereich der Längsnaht 35 erfolgt vorzugsweise durch Verschweißen, insbesondere durch Ultraschallschweißen, und/oder auf jede sonstige geeignete Art und Weise, beispielsweise durch Heißsiegeln, Kleben o. dgl.

Gemäß der Ausführungsvariante nach Fig. 8b ist es auch möglich, Längsränder des die Wandung 25 bildenden Materialstücks durch Überlappen zu einer Längsnaht 35 zu verbinden. In diesem Fall wird also ein Teil der Außenseite im Bereich der Überlappung mit der Innenseite des überlappenden Materialstückbereichs verbunden.

Bei der Ausführungsvariante gemäß Fig. 8c werden die Längskanten des die Wandung 25 bildenden Materialstücks zumindest im wesentlichen auf Stoß miteinander verbunden, um eine Längsnaht 35 zu bilden, und zwar vorzugsweise durch einen zusätzlichen Längs- oder Materialstreifen 38, also vorzugsweise durch nahtloses Längsverschweißen unter Verwendung von zusätzlichem Material (hierbei kann es sich um beispielsweise um das gleiche Material wie eine Innenbeschichtung der Wandung 25 oder um ein anderes, sich damit verbindendes Material handeln).

Der Fluidraum 4 bzw. die Wandung 25 kann alternativ auch ohne längsseitige Verbindung aus einem entsprechenden Endlosschlauchmaterial o. dgl., vorzugsweise aus einem schlauchförmig geblasenen oder extrudierten Material hergestellt oder gebildet werden.

Es ist anzumerken, daß das Reservoir 3 bzw. der vorzugsweise beutelartige Fluidraum 4 grundsätzlich bzw. bevorzugt auch so ausgebildet werden kann, wie in der WO 99/43571 A1 beschrieben.

Insbesondere können das vorschlagsgemäße Reservoir 3 und die vorliegende Erfindung generell auch bei den Zerstäubern bzw. Inhalern, die in den nachfolgend genannten Druckschriften beschrieben sind oder auf deren Prinzipien beruhen, verwendet werden: EP 1 236 517, EP 1 561 484, EP 1 562 094, EP 1 604 701, JP 2004-0283245, JP 2004-249208, JP 2004-283244, JP 2005-058421, US 2002/0153006, US 2003/0100964, US 2003/0127538, US 2004/0163646, US 2005/0034723, US 2005/0133029, US 2005/0172957, US 2005/0224076, US 2005/0268911, US 5,915,378, WO 03/041774, WO 2004/022128, WO 2004/039442 und WO 2004/078244.

Vorzugsweise handelt es sich bei dem Fluid 2 um eine Flüssigkeit, wie bereits erwähnt, insbesondere um eine wäßrige oder ethanolische Arzneimittelformulierung. Es kann sich jedoch auch um eine sonstige Arzneimittelformulierung, eine Suspension oder dgl. oder auch um Partikel oder Pulver handeln.

Nachfolgend werden bevorzugte Bestandteile, Verbindungen und/oder Formulierungen des vorzugsweise medizinischen Fluids 2 aufgeführt. Wie bereits erwähnt, kann es sich um wäßrige oder nicht wäßrige Lösungen, Mischungen, ethanolhaltige oder lösungsmittelfreie Formulierungen o. dgl. handeln. Besonders bevorzugt sind im Fluid 2 enthalten:

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetikum dar, kombiniert mit einem Anticholinergikum, Corticosteroide, PDE4-Inhibitore, EGFR-Hemmern oder LTD4-Antagonisten,
- W stellt ein Corticosteroid dar, kombiniert mit einem PDE4-Inhibitor, EGFR-Hemmern oder LTD4-Antagonisten
- W stellt einen PDE4-Inhibitor dar, kombiniert mit einem EGFR-Hemmer oder LTD4-Antagonisten
- W stellt einen EGFR-Hemmer dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8- {2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)=ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 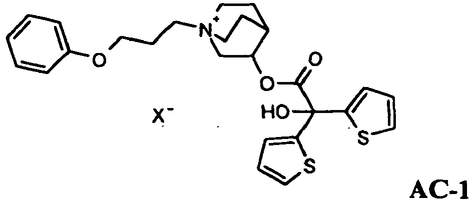 worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 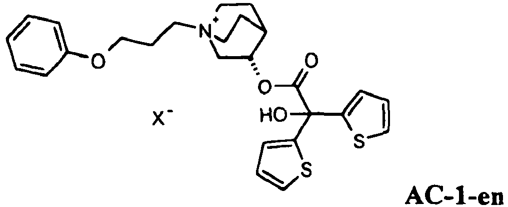 enthalten, worin X - die vorstehend genannten Bedeutungen aufweisen kann. Weiterhin bevorzugte Anticholinergika sind ausgewählt aus den Salzen der Formel AC-2 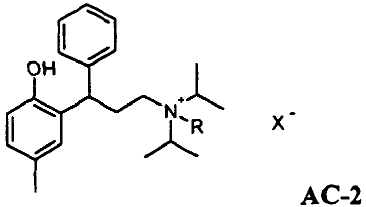 worin R entweder Methyl oder Ethyl bedeuten und worin X - die vorstehend genannte Bedeutungen aufweisen kann. In einer alternativen Ausführungsform kann die Verbindung der Formel AC-2 auch in Form der freien Base AC-2-base vorliegen.

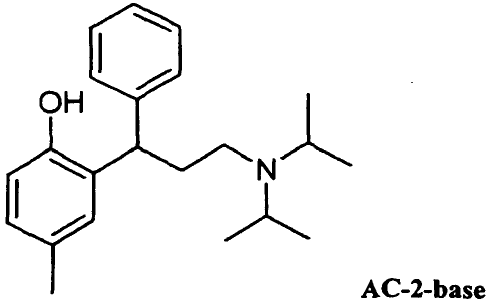

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester- Methobromid
- 9-Methyl-xanthen-9-earbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H-*pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxychinazolin
- 4-[(3-Chlor-4-fluor-phenyl)amino]-6-(2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-y1)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6- {cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

### Bezugszeichenliste

| | | | |
|---|---|---|---|
| 1 | Zerstäuber | 24 | Verschluß |
| 2 | Fluid | 25 | Wandung |
| 3 | Reservoir | 25a | Ringbereich |
| 4 | Fluidraum | 26 | erstes Verschlußteil |
| 5 | Druckerzeuger | 26a | Ringnut |
| 6 | Halterung | 26b | Anschlußbereich |
| 7 | Antriebsfeder | 26c | Einfüllöffnung |
| 8 | Sperrelement | 27 | zweites Verschlußteil |
| 9 | Förderrohr | 27a | Septum |
| 10 | Rückschlagventil | 27b | Einführöffnung |
| 11 | Druckkammer | 27c | Ringwulst |
| 12 | Austragsdüse | 28 | Versiegelung |
| 13 | Mundstück | 29 | Verbindungsbereich |
| 14 | Aerosol | 30 | Sperre |
| 15 | Zuluftöffnung | 31 | Anlagebereich |
| 16 | Gehäuseoberteil | 32 | Winkel |
| 17 | Innenteil | 33 | Achse |
| 17a | oberes Teil des Innenteils | 34 | Quernaht |
| 17b | unteres Teil des Innenteils | 35 | Längsnaht |
| 18 | Gehäuseteil (Unterteil) | 36 | Breite |
| 19 | Halteelement | 37 | Abstand |
| 20 | Feder (im Gehäuseunterteil) | 38 | Materialstreifen |
| 21 | Reservoirboden | 39 | Klebebereich |
| 21a | Belüftungsöffnung | 40 | Einfaltung |
| 22 | Anstechelement | 41 | Teil der Quernaht |
| 23 | Außenhülle | 42 | Teil der Quernaht |
| 23a | Vorsprung | | |

## Patentansprüche

1. Reservoir (3) für einen Zerstäuber (1) oder Inhalator, mit einem Fluidraum (4) für ein Fluid (2), Insbesondere eine Arzneimittelformulierung, und mit einem den Fluidraum (4) verschließenden Verschluß (24), der ein erstes Verschlußteil (26) und ein zweites Verschlußteil (27) aufweist, wobei die beiden Verschlußteile (26, 27) in einem Verbindungsbereich (29) fluiddicht miteinander verbunden sind,
wobei
der Verschluß (24) eine fluidseitig bezüglich des Verbindungsbereichs (29) angeordnete Sperre (30) zum Verhindern des Eindringens von beim verbinden der Verschlußteile (26, 27) potentiell entstehenden Partikeln oder sonstigen Verschmutzungen in das Fluid (2) und/oder in den Fluidraum (4) aufweist,
**dadurch gekennzeichnet, dass**
die Sperre (30) vom Verbindungsbereich (29) axial und radial beabstandet und radial umlaufend konzentrisch zum Verbindungsbereich (29) angeordnet ist, wobei
die Sperre (30) durch einen Paßsitz der beiden Verschlußteile (26, 27) gebildet ist, wobei die Verschlußteile (26, 27) in einem Auflagebereich (31) konisch ineinandergreifen und wobei beide Verschlußteile (26, 27) im Auflagebereich (31) zu einer Längsachse des Reservoirs (3) geneigt sind.

2. Reservoir nach Anspruch 1, **dadurch gekennzeichnet, dass** die Verschlußteile (26, 27) im Verbindungsbereich (29) miteinander verschweißt sind, insbesondere wobei die Verschlußteile (26, 27) durch Material des ersten und/oder zweiten Verschlußteils (26, 27) und/oder durch zusätzlich aufgebrachtes bzw. vorgesehenes Material, besonders bevorzugt in Form von Pulver oder eines Rings, miteinander im Verbindungsbereich (29) verbunden bzw. verschweißt sind.

3. Reservoir nach Anspruch 1 oder 2, **dadurch gekennzeichnet, dass** der Verbindungsbereich (29) umlaufend oder ringförmig ausgebildet ist.

4. Reservoir nach einem der voranstehenden Ansprüche, **dadurch gekennzeichnet, dass** der Verbindungsbereich (29) den Fluidraum (4) nach dem Einfüllen des Fluids (2) gasdicht verschließt.

5. Reservoir nach einem der voranstehenden Ansprüche, dass die Sperre (30) nur durch die Verschlußteile (26, 27) gebildet ist oder dass die Sperre (30) durch eine trichterförmige Anordnung gebildet ist.

6. Reservoir nach einem der voranstehenden Ansprüche, **dadurch gekennzeichnet, dass** der Fluidraum (4) eine flexible oder verformbare Wandung (25) aufweist, so daß der Fluidraum (4) bei der Entnahme von Fluid (2) kollablert und/oder einen verformbaren innenbehälter bildet

7. Reservoir nach Anspruch 6, **dadurch gekennzeichnet, dass** das erste Verschlußteil (26) an die Wandung (25) insbesondere durch Warmformen angeformt oder auf sonstige Weise damit fest verbunden ist, insbesondere wobei die Wandung (25) nur mit dem ersten Anschlußteil (26) fest oder unlösbar verbunden ist.

8. Reservoir nach einem der vorangegangenen Ansprüche, **dadurch gekennzeichnet, dass** der Auflagebereich (31) mit einem Winkel (32) geneigt zur Längsachse verläuft, wobei der Winkel (32) vorzugsweise etwa 15° beträgt.

9. Reservoir nach einem der vorangegangenen Ansprüche, **dadurch gekennzeichnet, dass** die Verbindung der beiden Verschlussteile (26,27) im Verbindungsbereich (29) im Wesentlichen axial ausgebildet ist und dass eine mit dem Verbinden der beiden Verschlussteile (26,27) einhergehende Bewegung in Verbindungsrichtung die Sperre (30) wirksam macht.

## Claims

1. Reservoir (3) for a nebuliser (1) or inhaler, having a fluid chamber (4) for a fluid (2), particularly a medicament formulation, and having a closure (24) that seals the fluid chamber (4), comprising a first closure member (26) and a second closure member (27), the two closure members (26, 27) being connected to each other in a fluidtight manner in an attachment region (29),
wherein
the closure (24) comprises a barrier (30) arranged on the fluid side with respect to the attachment region (29), to prevent the ingress of any particles potentially produced during the attachment of the closure members (26, 27) or other contaminants into the fluid (2) and/or into the fluid chamber (4),
**characterised in that**
the barrier (30) is axially and radially spaced from the attachment region (29) and is arranged in a radially circumferential manner, concentrically with respect to the attachment region (29),
the barrier (30) being formed by a press fit of the two closure members (26, 27), the closure members (26, 27) engaging conically in one another in an abutment region (31) and the two closure members (26, 27) being inclined in the abutment region (31) with respect to a longitudinal axis of the reservoir (3).

2. Reservoir according to claim 1, **characterised in that** the closure members (26, 27) are welded to each other in the attachment region (29), the closure members (26, 27) being attached or welded to each other by material of the first and/or second closure member (26, 27) and/or by material that is additionally applied or provided, particularly preferably in the form of powder or a ring.

3. Reservoir according to claim 1 or 2, **characterised in that** the attachment region (29) is of circumferential or annular configuration.

4. Reservoir according to one of the preceding claims, **characterised in that** the attachment region (29) seals the fluid chamber (4) in a gastight manner after the fluid (2) has been introduced.

5. Reservoir according to one of the preceding claims, **characterised in that** the barrier (30) is formed only by the closure members (26, 27) or **in that** the barrier (30) is formed by a funnel-shaped arrangement.

6. Reservoir according to one of the preceding claims, **characterised in that** the fluid chamber (4) has a flexible or deformable wall (25), so that when fluid (2) is taken out the fluid chamber (4) collapses and/or forms a deformable inner container.

7. Reservoir according to claim 6, **characterised in that** the first closure member (26) is moulded onto the wall (25) particularly by thermoforming or is firmly attached thereto by some other method, while in particular the wall (25) is firmly or non-releasably attached only to the first closure member (26).

8. Reservoir according to one of the preceding claims, **characterised in that** the abutment region (31) extends at an inclined angle (32) relative to the longitudinal axis, the angle (32) preferably being about 15°.

9. Reservoir according to one of the preceding claims, **characterised in that** the attachment of the two closure members (26, 27) in the attachment region (29) is of substantially axial configuration and **in that** a movement in the direction of attachment accompanying the attachment of the two closure members (26, 27) renders the barrier (30) operational.

## Revendications

1. Réservoir (3) destiné à un pulvérisateur (1) ou à un inhalateur, comprenant un espace pour fluide (4) destiné à un fluide (2), en particulier à une formulation de médicament, et comprenant un système de fermeture (24) fermant l'espace pour fluide (4), lequel système de fermeture présente une première partie de système de fermeture (26) et une deuxième partie de système de fermeture (27), les deux parties de système de fermeture (26, 27) étant reliées l'une à l'autre de manière étanche aux fluides dans une zone de liaison (29),
le système de fermeture (24) présentant un dispositif de blocage (30) disposé côté fluide par rapport à la zone de liaison (29), servant à empêcher l'intrusion dans le fluide (2) et/ou dans l'espace pour fluide (4) de particules se formant potentiellement lors de la liaison des parties de système de fermeture (26, 27) ou d'autres saletés éventuelles,
**caractérisé en ce que**
le système de blocage (30) est disposé de manière espacée axialement et radialement de la zone de liaison (29) et en périphérie radialement de manière concentrique par rapport à la zone de liaison (29),
le système de blocage (30) étant formé par un siège ajusté des deux parties de système de fermeture (26, 27), les parties de système de fermeture (26, 27) s'imbriquant l'une dans l'autre de manière conique dans une zone d'appui (31) et les deux parties de système fermeture (26, 27) étant inclinées dans la zone d'appui (31) par rapport à un axe longitudinal du réservoir (3).

2. Réservoir selon la revendication 1, **caractérisé en ce que** les parties de fermeture (26, 27) sont soudées l'une à l'autre dans la zone de liaison (29), les parties de système de fermeture (26, 27) étant en particulier reliées ou soudées l'une à l'autre dans la zone de liaison (29) par un matériau de la première et/ou de la deuxième partie de système de fermeture (26, 27) et/ou par un matériau appliqué ou prévu en supplément, de manière particulièrement préférée se présentant sous la forme de poudre ou sous la forme d'un anneau.

3. Réservoir selon la revendication 1 ou 2, **caractérisé en ce que** la zone de liaison (29) est réalisée de manière périphérique ou de manière à présenter une forme annulaire.

4. Réservoir selon l'une quelconque des revendications précédentes, **caractérisé en ce que** la zone de liaison (29) ferme de manière étanche aux gaz l'espace pour fluide (4) après le remplissage avec le fluide (2).

5. Réservoir selon l'une quelconque des revendications précédentes, **caractérisé en ce que** le système de blocage (30) est formé uniquement par les parties de système de fermeture (26, 27), ou **en ce que** le système de blocage (30) est formé par un ensemble présentant une forme d'entonnoir.

6. Réservoir selon l'une quelconque des revendications précédentes, **caractérisé en ce que** l'espace pour fluide (4) présente une cloison (25) flexible ou déformable, de sorte que l'espace pour fluide (4) s'effondre lors du retrait du fluide (2) et/ou forme un récipient intérieur déformable.

7. Réservoir selon la revendication 6, **caractérisé en ce que** la première partie de système de fermeture (26) est formée au niveau de la cloison (25) en particulier par des formages à chaud ou est reliée à ladite cloison de manière solidaire d'une autre manière, la cloison (25) étant reliée en particulier de manière solidaire ou de manière inamovible uniquement à la première partie de raccordement (26).

8. Réservoir selon l'une quelconque des revendications précédentes, **caractérisé en ce que** la zone d'appui (31) s'étend de manière inclinée selon un angle (32) donné par rapport à l'axe longitudinal, l'angle (32) étant égal de préférence à environ 15°.

9. Réservoir selon l'une quelconque des revendications précédentes, **caractérisé en ce que** la liaison des deux parties de système de fermeture (26, 27) est réalisée essentiellement de manière axiale dans la zone de liaison (29), et **en ce qu'**un déplacement dans la direction de liaison allant de pair avec la liaison des deux parties de système de fermeture (26, 27) rend le dispositif de blocage (30) actif.
